# EUROPÄISCHE PATENTSCHRIFT

(11) **EP 0 799 124 B1**
(45) Veröffentlichungstag und Bekanntmachung des Hinweises auf die Patenterteilung: **16.08.2001**
(21) Anmeldenummer: 95942172.8
(22) Anmeldetag: 18.12.1995
(51) Int. Cl.: B29C 70/40, B29C 70/08, B29K 101/12, B29L 1/00, A61B 17/68

(54) **VERFAHREN ZUR HERSTELLUNG VON BAUTEILEN AUS FASERVERSTÄRKTEN THERMOPLASTEN SOWIE NACH DEM VERFAHREN HERGESTELLTER BAUTEIL**
PROCESS FOR MANUFACTURING COMPONENTS MADE OF FIBRE-REINFORCED THERMOPLASTIC MATERIALS AND COMPONENTS MANUFACTURED BY THIS PROCESS
PROCEDE DE FABRICATION DE COMPOSANTS EN MATIERES THERMOPLASTIQUES RENFORCEES PAR DES FIBRES ET COMPOSANTS FABRIQUES SELON CE PROCEDE

(30) Priorität: 19.12.1994 DE 4445305; 19.12.1994 DE 4445307
(43) Veröffentlichungstag der Anmeldung: 08.10.1997
(73) Patentinhaber: Sepitec Foundation, 9490 Vaduz (LI)
(72) Erfinder: LOHER, Urs, CH-9500 Wil (CH); MAYER, Jörg, CH-8634 Hombrechtikon (CH); TOGNINI, Roger, D-79771 Klettgau (DE); WEGENER, Thomas, CH-9042 Speicher (CH); WINTERMANTEL, Erich, 5422 Fislisbach (CH)
(74) Vertreter: Menges, Rolf, Dipl.-Ing.
(86) Internationale Anmeldenummer: EP9504992
(87) Internationale Veröffentlichungsnummer: WO9619336

(56) Entgegenhaltungen:
- EP-A- 0 373 294
- EP-A- 0 376 472
- WO-A-89/00649
- WO-A-91/02906
- WO-A-94/07425
- DE-A- 3 739 582
- US-A- 3 859 409
- US-A- 4 655 777
- PATENT ABSTRACTS OF JAPAN vol. 009, no. 087 (M-372), 17.April 1985 & JP,A,59 215821 (MITSUBISHI JUKOGYO KK), 5.Dezember 1984,

## Beschreibung

Die Erfindung betrifft ein Verfahren zur Herstellung von Bauteilen aus faserverstärkten Thermoplasten. wobei vorerst ein aus Kurz-, Lang- und/oder Endlosfaser und einem Thermoplast gebildeter Rohling vorgefertigt und dieser Rohling in einem Warmumformverfahren unter Druck in einer Negativform in die endgültige Gestalt des Bauteiles gebracht wird, ein Verfahren zur Herstellung von auf Zug. Biegung und/oder Torsion beanspruchten Bauteilen aus faserverstärkten Thermoplasten, wobei vorerst ein mit einem Faseranteil von mehr als 50 Vol-% und unter zumindest überwiegendem Einsatz von Endlosfasern und einem Thermoplast gebildeter Rohfing vorgefertigt und dieser Rohling in einem Warmumformverfahren unter Druck in einer Negativform in die endgültige Gestalt des Bauteiles gebracht wird, sowie einen Bauteil aus faserverstärkten Thermoplasten, hergestellt nach einem dieser Verfahren.

Bauteile aus faserverstärkten Thermoplasten werden meist als Verbindungselemente eingesetzt. Durch diese Bauteile sollen z.B. Metallschrauben ersetzt werden. Gerade beim Einsatz in der Medizintechnik, also beispielsweise bei Knochenschrauben, sind Schrauben aus faserverstärkten Thermoplasten wesentlich besser geeignet als Metallschrauben da sie zum Knochen strukturkompatibel sind, keine Probleme mit der Korrosionsfestigkeit auftreten. das Gewicht gegenüber Metallschrauben verringert werden kann und die üblichen medizinischen Untersuchungsmethoden im Gegensatz zum Einsatz von Metall nicht beeinträchtigt werden.

Es sind schon Schrauben und. Gewindestäbe aus faserverstärkten thermoplastischen Kunststoffen bekannt geworden, wobei die Schraubenrohlinge entweder durch Coextrusion oder durch ein Mehrkomponenten-Spritzgießverfahren hergestellt werden. Bei dieser bekannten Ausführung (DE-A-40 16 427) werden als Ausgangsmaterial kreisrunde Vollstangen, die mittels Coextrusion hergestellt werden, eingesetzt. Für den Kernbereich wird in einem Extruder thermoplastisches Granulat mit 5-10 mm Langfasern aufbereitet, für den äußeren Bereich in einem zweiten Extruder thermoplastisches Granulat mit Kurzfasern aufbereitet. Somit ist ein Ausgangsmaterial gegeben, bei welchem eine koaxiale Anordnung mit inneren Langfasern und äußeren Kurzfasern vorhanden ist. Die Langfasern im inneren Kernbereich sind durch einen Extrusionsfließvorgang vorwiegend axial ausgerichtet, die Kurzfasern im äußeren Bereich übertragen Abscherkräfte in den Gewindegängen. Die Gewindegänge werden durch anschließendes Kaltumformen. z.B. mittels Gewinderollköpfen oder -maschinen hergestellt. Eine solche Kaltumformung wird durch den Einsatz von Kurzfasern möglich gemacht, doch ergeben sich gerade aus der Anordnung solcher Kurzfasern im Gewindebereich verringerte Festigkeitswerte.

Bei einem Verfahren gemäß der DE-T2-68919466 wird ein Rohling in eine teilbare Form eingelegt und in dieser verformt. Der Rohling wird kalt in eine Formkavität eingelegt, erwärmt, aufgeweitet und abgekühlt. Es ist daher nur ein Umformen in beschränktem Maße möglich, wobei außerdem eine Beeinflussung der Ausrichtung der Fasern praktisch unmöglich oder zumindest nicht vorherbestimmbar möglich ist.

Bei einem Verfahren gemäß der US-A-3 859 409 wird der Rohling zwar aus einer Vorkammer in die eigentliche Formkavität gedrückt, diese weist jedoch einen größeren Durchmesser als der Rohling auf. Auch bei diesem bekannten Verfahren wird der Rohling lediglich in seinen Randbereichen und in seinen Endbereichen verformt.

Aus der EP-A-0 376 472 ist ein thermoplastisches Verbundplattenmaterial bekannt, das quasi isotrop ausgebildet ist und leicht in eine Form gepreßt werden kann, um einen Verbundgegenstand von komplizierter Gestalt herzustellen, wie beispielsweise Golfschlägerköpfe.

In der EP 0 373 294 A3 ist ein Verfahren unter anderem zur Herstellung von schraubenförmigen, faserverstärkten Befestigungselementen beschrieben gemäß dem einleitenden Teil des Anspruchs 1. Hierbei wird zur Herstellung eines Endprodukts ein stangenförmig ausgebildeter Rohling aus faserverstärktem Kunststoff in eine Negativform eingelegt, dort erwärmt und anschließend der Gestalt der Negativform unter Druck angepaßt. Hierbei handelt es sich um ein Verfahren, das dem Gewinderollen oder Gesenkschmieden verwandt ist, da nur eine partielle Umformung des Rohlings stattfindet. Bei den Fasern des Rohlings aus faserverstärktem Kunststoff handelt es sich um Endlosfasem, die nur im äußeren Bereich des Rohlings in radialer Richtung entlang der Negativform verändert werden. Eine axiale Faserausrichtung ist bei diesem Verfahren nur am äußeren Rand gegeben, da sich die Fasern dort radial an die Negativform anlegen.

Die vorliegende Erfindung hat sich nun zur Aufgabe gestellt, ein Verfahren zur Herstellung eines Bauteiles aus faserverstärkten Thermoplasten zu schaffen, mit welchem eine optimale Anpassung an den Einsatzzweck eines Bauteiles möglich ist. Weiter ist es Aufgabe der Erfindung, einen nach diesem Verfahren hergestellten Bauteil zu schaffen, mit welchem sich in besonderer Weise die Krafteinleitung und -verteilung sowie die Steifigkeit an die Beschaffenheit des mit dem Bauteil zusammenwirkenden Körpers anpassen lassen.

Das erste erfindungsgemäße Verfahren sieht demnach vor, daß der Rohling zunächst in einer Erwärmungsstufe auf Umformtemperatur erwärmt und dann durch Fließpressen in die Negativform eingepreßt wird. Die Fasern, welche über den ganzen Querschnitt des Rohlings verteilt sind, werden durch das nachfolgende Fließpreßverfahren ganz gezielt steuerbar orientiert und verteilt. Die Faserorientierung und Faserverteilung und somit die mechanischen Eigenschaften eines nach diesem Verfahren hergestelften Bauteiles können somit speziell charakterisiert und zu den Prozeßparametern des Herstellverfahrens in bezug gebracht werden. Durch das Fließpressen kann zusätzlich die Faserorientierung gesteuert werden, so daß auch auf die Länge eines entsprechenden Bauteiles unterschiedliche Festigkeitswerte erzielbar sind.

Vorteilhafte Ausgestaltungen der Erfindung bilden die Gegenstände der unteransprüche.

In einer bevorzugten Ausführung dieses Verfahrens wird unter Einsatz von mehr als 50 Vol-% Endlosfasern der Rohling zunächst in einer Erwärmungsstufe auf Umformtemperatur erwärmt und dann durch Fließpressen in die Negativform eingepreßt. Gerade beim Einsatz von einer großen Dichte von Endlosfasern sind die Steifigkeit und die Festigkeit eines herzustellenden Bauteiles ganz gezielt steuerbar. Insbesondere bei kompliziert geformten Bauteilen wirken sich die exakte Vorausberechenbarkeit des optimalen Faserverlaufes und der oprimalen Faserdichte in einem bestimmten Bereich vorteilhaft aus.

So wird weiter vorgeschlagen, daß der Rohling als Stangenmaterial vorgefertigt und vor dem Warmumformverfahren in für den endgültigen Bauteil erforderliche Längen zugeschnitten wird. Die für den endgültigen Bauteil notwendigen Materialstücke werden aus dem vorgefertigten Stangenmaterial abgetrennt und anschließend dem Warmumformverfahren zugeführt. Es ist also eine dem Fließpreßverfahren bei Metallteilen ähnliche Vorgangsweise vorgesehen.

Durch den Einsatz von Endlosfasern deren Länge wenigstens der Länge des Rohlings für den endgültigen Bauteil entspricht, können noch verbesserte Steifigkeiten und Festigkeiten erzielt werden.

Es wäre auch denkbar, daß ein Rohling aus in dessen Längsrichtun verlaufenden Schichten unterschiedlicher Faserorientierung umgeformt wird. Es können also durch vorteilhalfte Ausgestaltung der erfindungsgemäßen Verfahren unzählige neue Einsatzgebiete erfaßt werden, da immer auf einen ganz speziellen Einsatzzweck hingearbeitet bei den herzustellenden Bauteilen eine exakt vorherbestimmbare Festigkeit und Steifigkeit erzielbar ist.

In diesem Zusammenhang ist es auch möglich, daß ein Rohling aus mehr als einem Polymerverbund, z.B. mit mehreren Schichten mit unterschiedlichem Matrixwerkstoff und unterschiedlicher Anordnung und/oder unterschiedlichem Vol%-Anteil und/oder unterschiedlichem Fasermaterial und/oder unterschiedlicher Länge der Fasern, umgeformt wird. Auch mit solchen Maßnahmen kann eine exakte Anpassung an die Enderfordernisse des herzustellenden Bauteiles erfolgen.

In diesem Zusammenhang kann es auch von Vorteil sein, wenn der Rohling durch ein Gegentaktfließpreßverfahren in den endgültigen Bauteil umgeformt wird. Der vom Stangenmaterial abgetrennte Rohling wird in einer entsprechenden Fließpreßform umgeformt. Es wird also das sogenannte Durchdrückverfahren nach DIN 8583 eingesetzt. Beim Gegentaktfließpreßverfahren wird der Rohling in mehrfachen Hin- und Herbewegungen in der Negativform in den endgültigen Bauteil umgeformt. Gerade bei der Herstellung von leisten- oder plattenförmigen Bauteilen wirkt sich dieses Verfahren besonderes positiv aus.

Gegenüber dem Fließpressen oder dem Gegentaktfließpressen von Metallteilen ist dabei als wesentliches Unterscheidungsmerkmal vorgesehen, daß beim Fließ- oder Gegentaktfließpreßverfahren der Rohling in einer Erwärmungsstufe auf eine Umformtemperatur von z.B. 350 - 450°C erwärmt und dann in die Negativform eingepreßt wird, wobei während einer Nachdruckphase eine Abkühlung unter die Glasübergangstemperatur des Thermoplasts von z.B. 143°C erfolgt. Für die Verarbeitung der faserverstärkten Thermoplaste wird das bei Metallteilen bekannte Fließpreßverfahren dahingehend verändert, als der Rohling nicht bei Raumtemperatur, sondern oberhalb der Schmelz- oder Erweichungstemperatur des Matrix-Werkstoffes umgeformt wird.

Weiter ist es vorteilhaft, daß beim Warmumformverfahren als Trennmittel Kohlenstoff oder Graphit eingesetzt wird. Eine solches Trennmittel wurde bei der Umformung von Thermoplasten bisher offensichtlich nicht eingesetzt. Hier ergibt sich der zusätzliche besondere Vorteil, daß z.B. Graphit im Gegensatz zu den sonst üblichen Beschichtungen oder Trennmitteln, welche für Kunststoffe eingesetzt werden, biokompatibel ist, so daß sich gerade Bauteile für den medizinischen Bereich dazu eignen. Weiter ist als vorteilhafte Ausgestaltung der erfindungsgemäßen Verfahren vorgesehen, daß ein Rohling aus mit Kohlenstoffasern verstärktem PAEK (Poly-Aryl-Ether-Ketone) verarbeitet wird. Es hat sich gezeigt, daß durch die Verwendung gerade eines solchen Werkstoffes die Zugfestigkeit des derart gefertigten Bauteiles im Schnitt etwa 30% unter der Zugfestigkeit vergleichbarer Stahl-Bauteile liegt. Für den Einsatzbereich solcher Bauteile aus faserverstärkten Thermoplasten ist dies aber eine mehr als ausreichende Festigkeit, da ja immer auch betrachtet werden muß, mit welchen Materialien ein solcher Bauteil zusammenwirken soll. Gerade beim Einsatz in der Medizintechnik, also z.B. bei Knochenschrauben, oder bei Platten- oder Schienenbauteilen ist eine entsprechend hohe Bruchkraft durchaus ausreichend, da solche Bauteile schon nahezu das Dreifache der verfügbaren Bruchkraft eines Knochens aufweisen.

Als weitere vorteilhalfte Augestaltung der beiden erfindungsgemäßen Verfahren wird ferner vorgesehen, daß bei zumindest einem Anteil an Fasern diese im Rohling achsparallel verlaufen. Denkbar ist aber auch, daß bei zumindest einem Anteil an Fasern diese eine Ausrichtung von 0 bis zu 90° aufweisen. Vor allem bei der Herstellung von langgestreckten Bauteilen, z.B. in Form einer Schraube oder eines streifenförmigen Montageteiles. ergeben sich dadurch besondere Anpassungsmöglichkeiten an die notwendigen Festigkeitsbereiche. Der Elastizitätsmodul von Schrauben, die aus Rohlingen mit achsparallel ausgerichteten Fasern hergestellt wurden, ist entsprechend höher, solche Schrauben sind also tendenziell steifer. Es hat sich gezeigt, daß durch den Einsatz eines Fließpreverfahrens eine Veränderung des Faserverlaufes gegenüber dem Faserverlauf im Rohling möglich ist, so daß durch die spezielle Faserorientierung im Rohling zusätzliche Anpassungsparameter möglich werden.

Als weitere vorteilhalfte Augestaltung der beiden erfindungsgemäßen Verfahren können Fasern eingesetzt, welche eine Länge von mehr als 3 mm aufweisen. Bei vielen bekannten faserverstärkten Thermoplasten zur Herstellung entsprechender Bauteile werden in der Regel Kurzfasern oder Langfasern eingesetzt. Der Einsatz von Endlosfasern mit dem hohen Faseranteil von mehr als 50 Vol-% ergibt im Zusammenhang mit dem Warmumformverfahren eine optimale Möglichkeit, die Festigkeitseigenschaft an jeder Stelle des zu fertigenden Bauteiles entsprechend zu steuern, so daß lokal gezielt eingestellte Steifigkeiten erreichbar sind.

Als weitere vorteilhalfte Augestaltung der beiden erfindungsgemäßen Verfahren werden die Fasern beim Fließpressen oberflächendeckend vom Matrixmaterial umschlossen Somit ist auch bei den dann endgültig durch das Fließpreß verfahren hergestellten Bauteilen keine Nachbearbeitung mehr nötig, da die gesamte Oberfläche praktisch bereits versiegelt ist.

Als weitere vorteilhalfte Augestaltung der beiden Verfahren nach der Erfindung ist es auch möglich, daß die Bauteile bei der Warmumformung eine zusätzliche Oberflächenversiegelung erhalten. Durch den Einfluß der Wärme im Umformwerkzeug oder entsprechende zusätzliche Mittel, z.B. Beschichtungen oder Trennmittel, kann eine zusätzliche Oberflächenversiegelung der fertigen Bauteile erzielt werden.

Durch die erfindungsgemäßen Verfahren ergeben sich verschiedene Möglichkeiten, den Herstellungsprozeß zu steuern. Ein nach den erfindungsgemåßen Verfahren hergestellter Bauteil ist daher gekennzeichnet durch Bereiche mit durch einen vorherbestimmten Verlauf der Fasern lokal vorherbestimmten Steifigkeiten und Festigkeiten, Die höchsten Zugfestigkeiten wurden beispielsweise bei Bauteilen erzielt, die bei hohen Umformgeschwindigkeiten und hohen Rohlingtemperaturen hergestellt wurden. Bei Berücksichtigung der Torsionsfestigkeit hingegen werden dann Maximalwerte erzielt, wenn vergleichsweise tiefe Umformtemperaturen und eine niedrige Umformgeschwindigkeit eingesetzt werden. Es werden also gerade bei der Herstellung von Bauteilen aus faserverstärkten Thermoplasten durch die erfindungsgemäßen Verfahren Möglichkeiten geschaffen, einen Bauteil für den speziellen Einsatzzweck anzupassen, wobei es auch durchaus möglich wäre, einen Arbeitsgang z.B. aus zwei oder mehr als zwei Stufen verschiedener Umformgeschwindigkeiten zusammenzusetzen.

Durch Anpassung an die Form und den Einsatz des Bauteiles kann ein vorherbestimmbarer Verlauf der Fasern bezogen auf die Längsrichtung, den Durchmesser, die Dicke, die Form des Bauteiles oder es können bei Durchbrechungen, Vertiefungen, Einbuchtungen oder dergleichen im Bauteil Bereiche unterschiedlicher Faserorientierung oder unterschiedlichen Faserverlaufes vorgesehen sein. Ein solcher Bauteil ist in besonderer Weise an einen speziellen Einsatzzweck anpaßbar. Es kann also bei einem solchen Bauteil die Krafteinleitung und -verteilung besser an die Beschaffenheit des mit diesem Bauteil zusammenwirkenden Körpers angepaßt werden. Dies gilt in besonderer Weise für die Medizintechnik, beispielsweise bei Knochenschrauben oder bei medizinischen Montageteilen und Befestigungsstreifen usw., aber auch bei anderen Anwendungen im Maschinenbau, im Elektro- oder. Elektronikeinsatz oder in der Bautechnik.

Deshalb ist auch vorteilhafter weise der Bauteil als Verbindungselement mit einem Angriffsende für ein Werkzeug und einen Gewindeschaft ausgeführt, wobei die Steifigkeit des Verbindungselementes durch unterschiedliche Faserorientierung vom Angriffsende zum freien Ende hin variiert. Gerade bei für den Knochenbereich einsetzbaren Bauteilen ist eine Anpassung an die natürliche Struktur eines Knochens möglich, so daß ein leichtes, amagnetisches, röntgentransparentes und biokompatibles Verbindungselement geschaffen werden kann. Im Gegensatz zu meist üblichen Metallschrauben kann durch die Anpassung der Faserstruktur und des Faserverlaufes ein echt wirksamer Bauteil geschaffen werden.

Weiter als vorteilhafter Ausgestaltung des erfindungsgemäßen Bauteils vorgeschlagen, daß die Fasern vom Angriffsende her bis über die unmittelbar daran anschließenden Gewindegänge zumindest annähernd parallel zur Mittelachse des Bauteiles verlaufen, wogegen die Fasern im restlichen Gewindeabschnitt oberflächennah der Gewindekontur in Achsrichtung des Bauteiles folgen, im Kernbereich dieses Abschnittes jedoch eine zum freien Ende hin zunehmend zufällig verteilte Faserorientierung vorgesehen ist. Daher ist gerade im Bereich des Angriffes des als Schraube ausgebildeten Bauteiles und im daran anschließenden Gewindeabschnitt die größte Festigkeit vorhanden, wogegen die in den Knocheninnenbereich hineingreifenden Gewindeabschnitte eine geringere Zugfestigkeit aufweisen, da ja gerade in diesem Bereich auch keine Zugkräfte aufgenommen werden könnten.

In weiterer Ausgestaltung des erfindungsgemäßen Bauteils ist es von Vorteil, daß die Steifigkeit des Bauteiles durch unterschiedliche Faserorientierung vom Angriffsende her gesehen zum freien Ende hin stufenförmig oder kontinuierlich abnimmt. Daher kann gerade durch den Faserverlauf, der sich durch die erfindungsgemäßen Herstellungsverfahren und natürlich auch durch die Umformgeschwindigkeit ergibt, eine exakte Anpassung an den Einsatzbereich des Bauteiles erzielt werden.

Weiter wird vorgeschlagen, daß im Bauteil wenigstens ein Sackloch oder eine Durchgangsöffnung. z.B. zum Einsatz eines Drehwerkzeuges oder zum Durchführen von Befestigungsmitteln, vorgesehen ist. Durch eine solche Anordnung ist es möglich, entsprechende Torsionskräfte beim Eindrehen eines solchen schraubenförmigen Bauteiles, im besonderen beim eventuell notwendigen Herausdrehen aufzubringen. Bei Durchgangsöffnungen oder dergleichen ergibt sich auch bei flachen Bauteilen eine vorteilhafte Ausführung, da z.B. der die Öffnung umschließende Bereich mit besonderer Faserorientierung verstärkt werden kann. In diesem Zusammenhang ist es vorteilhaft, wenn das Sackloch oder die Durchgangsöffnung bei der Herstellung des Bauteiles eingeformt ist. Gerade bei einem Warmumformverfahren ergeben sich hier besondere zusätzliche Möglichkeiten, um eben in einem Umformverfahren auch gleich entsprechende Sacklöcher oder Durchgangsöffnungen für Drehwerkzeuge vorzusehen.

Ein besonderer Einsatzbereich für einen erfindungsgemäßen Bauteil ergibt sich dann, wenn der Bauteil als fur den medizinischen Einsatz strukturkompatible Corticalis oder Spongiosa-Schraube ausgebildet ist.

Ein weiteres Ausführungsbeispiel für einen Bauteil sieht vor, daß dieser als streifen- oder plattenförmiger Montageteil mit einer oder mehreren Durchgangsöffnungen und/oder über die Längsund/oder Seitenbegrenzungen vorstehenden Abschnitten ausgebildet ist, wobei die Steifigkeit und Festigkeit über dessen ganze Länge und/oder Breite und/oder Durchmesser vorherbestimmt ist. Es läßt sich also über die erfindungsgemäßen Verfahren jede Art Bauteil besonderer Gestaltung herstellen, wobei eine Anpassung an die notwendige Festigkeit und Steifigkeit auch ganz bestimmter Abschnitte möglich ist. da eben die Faserorientierung und -dichte vorherbestimmt werden kann.

In diesem Zusammenhang ist als vorteilhafte Ausgestaltung vorgesehen, daß der als Montageteil ausgebildete Bauteil durch dichtere Anordnung von Fasern im Bereich von Durchgangsöffnungen und/oder vorstehenden Abschnitten in diesen üblicherweise geschwächten Zonen die gleiche Festigkeit und Steifigkeit aufweist wie in anderen Bereichen des Bauteiles. Jeder Bauteil kann also so ausgelegt werden, daß er keine geschwächten Zonen mehr aufweist, so daß auch für ganz spezielle Einsatzzwecke die in allen Abschnitten notwendige Festigkeit und Steifigkeit erzielbar ist.

Für eine solcherart anpaßbare Festigkeit und Steifigkeit ist es daher geradezu optimal, wenn der Bauteil als Osteosyntheseplatte z.B. für den Einsatz mit einer Corticalis- oder Spongiosa-Schraube ausgebildet ist.

Ausführungsbeispiele der Erfindung werden in der nachstehenden Beschreibung anhand des in der Zeichnung dargestellten Ausführungsbeispieles noch näher erläutert. Es zeigen:
Fig.1 einen Abschnitt eines stabförmigen Rohlings, teilweise aufgeschnitten dargestellt, um eine 0°-Orientierung von eingeschlossenen Endlosfasern aufzuzeigen;
Fig.2 einen Bauteil in Form einer Schraube, wobei eine schematische Darstellung der Faserorientierungsverteilung in der Schraube eingezeichnet ist;
Fig.3 ein Diagramm über den Verlauf der Steifigkeit bezogen auf die Länge des als Verbindungselement vorgesehenen Bauteiles;
Fig.4 eine Prinzip-Skizze eines möglichen Schmelzfließpreßwerkzeuges mit Temperaturzonen zur Herstellung des Bauteiles;
Fig.5 eine schematische Darstellung einer weiteren Ausführungsform eines Fließpreßwerkzeuges,
Fig.6 eine Prinzipskizze für eine Herstellung eines Bauteiles im Gegentaktfließpreßverfahren;
Fig.7 eine Draufsicht auf einen in einem Gegentaktfließpreßverfahren hergestellten Bauteil, der speziell als Osteosyntheseplatte einsetzbar ist.

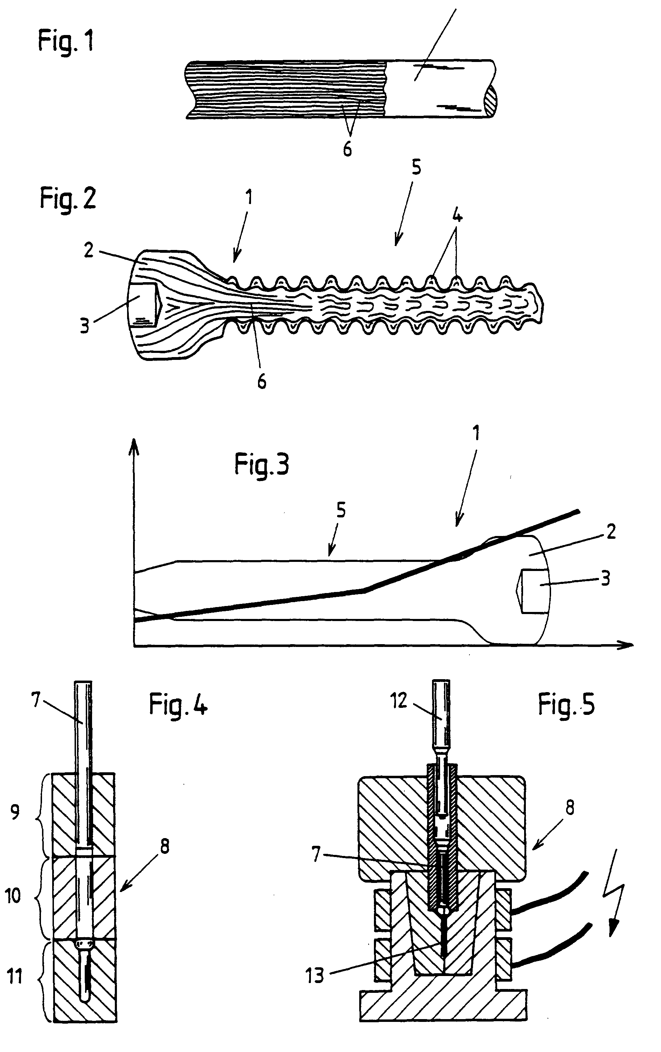

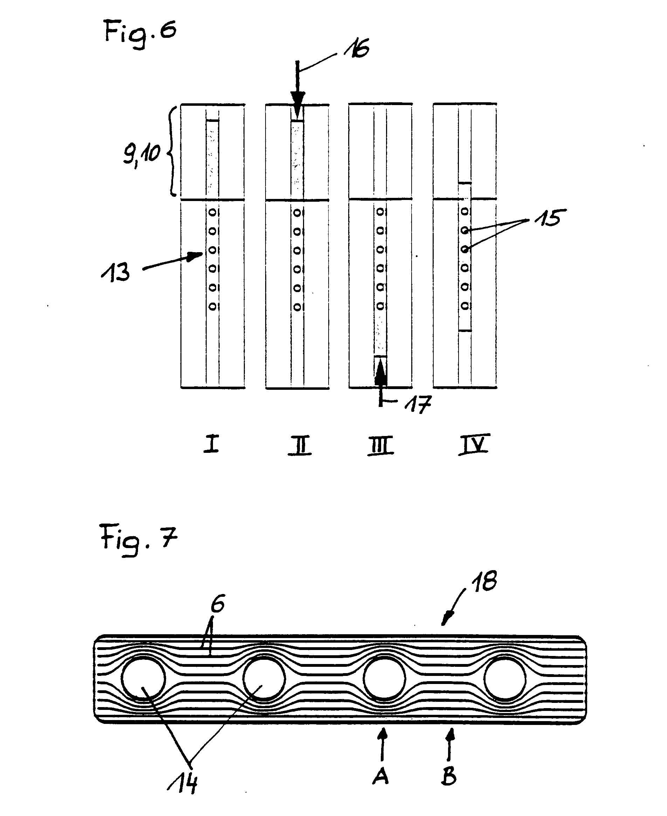

Bei der nachfolgenden Erläuterung der erfindungsgemäßen Verfahren sowie des nach den Verfahren hergestellten Bauteiles wird davon ausgegangen, daß der Bauteil (gemäß den Figuren 1 bis 5) ein Verbindungselement, insbesondere eine Schraube ist, die speziell in der Medizintechnik, also beispielsweise als Corticalis- oder Spongiosa-Schraube, eingesetzt wird, oder daß der Bauteil (gemäß den Figuren 6 und 7) ein Montageteil ist, insbesondere eine Osteosyntheseplatte für das Zusammenwirken mit einem vorstehend genannten Verbindungselement. Natürlich sind auch andere Bauteile hier mitumfaßt, welche aus faserverstärkten Thermoplasten bestehen und durch die erfindungsgemäßen Verfahren hergestellt werden. Die Anwendung solcher Bauteile ist dabei nicht nur auf die Medizintechnik beschränkt. Es ist durchaus denkbar, solche Bauteile auch in anderen Anwendungsbereichen, z.B. im Maschinenbau, in der Elektrotechnik, in der Raumfahrttechnik, im Hoch- oder Tiefbau usw., einzusetzen. Die Bauteile müssen auch nicht immer notgedrungen in Form von Verbindungselementen (Schrauben) hergestellt sein, sondem können auch als Bauteile mit ganz anderen konstruktiven Ausgestaltungen, wie z.B. Schienen oder Platten, eingesetzt werden. So wäre es beispielsweise denkbar, die in der Regel wohl nicht als selbstbohrende Schrauben ausführten Bauteile aus faserverstärkten Thermoplasten mit einem entsprechenden Bohrteil zu bestücken, der gegebenenfalls ebenfalls aus biokompatiblem Material gefertigt ist oder aber nach dem Bohrvorgang leicht entfernt werden kann. Unter Umständen ist eine solche Entfernung bei verschiedenen Anwendungsbereichen gar nicht notwendig. Das Beispiel wird auch anhand eines faserverstärkten Thermoplastes erläutert, welches mit Endlosfasern mit einem Volumenanteil von mehr als 50% gefertigt ist. Mit den erfindungsgemäßen Verfahren sind aber genauso vorteilhaft faserverstärkte Thermoplaste verarbeitbar, welche nur Kurzfasern oder Langfasern oder aber Kombinationen von Anteilen an Kurz-, Lang- und/oder Endlosfasern enthalten. Die erfindungsgemäßen Verfahren Lassen sich auch bei einem Faseranteil im Rohling von unter 50 Vol-% erfolgreich einsetzen.

Das in der Zeichnung dargestellte Verbindungselement in Form einer Schraube 1 besteht im wesentlichen aus einem Kopf 2, einem Angriff 3 für die Krafteinleitung von einem Drehwerkzeug her und einem mit einem Gewinde 4 versehenen Schaft 5. Wie gerade aus der Fig. 2 der Zeichnung ersichtlich ist, geht es bei der Schraube 1 im besonderen um den Verlauf der Endlosfasern 6. Durch gezielt lokal gerichtete Fasern innerhalb der Struktur verfügt die Schraube 1 über lokal gezielt eingestellte Steifigkeiten. Dadurch läßt sich gerade bei der Verwendung als Corticalis-Schraube die Steifigkeit an die natürliche Struktur eines Knochens anpassen. Durch die Wahl eines Verbundes von Thermoplasten mit Kohlenstoffasern läßt sich ein leichtes, röntgentransparentes und biokompatibles Verbindungselement schaffen. Der besondere Vorteil einer solchen Schraube liegt darin, daß die Steifigkeiten und die Steifigkeitsgradienten besser an die natürliche Struktur des Knochens angepaßt sind als bei herkömmlichen Metallschrauben. Durch die Faserstruktur wird eine bessere Kraftverteilung gewährleistet, d.h., es sind nicht mehr nur die ersten drei Gewindegänge tragend. Des weiteren beeinträchtigt das Verbindungselement die üblichen medizinischen Untersuchungsmethoden nicht, da es amagnetisch und röntgentransparent ist. Dies ist ein besonderer Nachteil herkömmlicher Metallimplantate, darunter auch Verbindungselemente. Sie können die Untersuchungsbefunde von modernen diagnostischen Methoden, wie z.B. Computertomographie oder Kernspintomographie. wertlos machen.

Durch das Nachstellverhalten des Verbindungselementes ist eine Lockerung erst nach längerer Zeit zu erwarten. Bei Ausbildung des Verbindungselementes als Corticalis-Schraube läßt sich die Schraube nach einem Überdrehen mit der verbleibenden Restfestigkeit wieder herausdrehen.

Wie schon ausgeführt, läßt sich das Verbindungselement im allgemeinen Maschinenbau in korrosiven Umgebungen und insbesondere dort einsetzen, wo hohe Festigkeiten, gezielte Festigkeiten bei geringerem Gewicht verlangt werden. Auch hier ist die Krafteinleitung über mehr als drei Gewindegänge ausschlaggebend.

Mit dem Kopf 2 der in Fig. 2 gezeigten Corticalis-Schraube können verschiedene weitere Elemente fixiert werden, z.B. eine Osteosyntheseplatte. Der Angriff 3 kann beispielsweise als Innensechskant ausgeführt sein. Es ist aber durchaus auch denkbar, andere Angriffs-oder Eingriffsformen zu wählen, z.B. eine Vierkantöffnung, eine Innensternöffnung oder einen Kreuzschlitz.

Eine Abwandlung des Fließpreßverfahrens, wie es aus der Metallbearbeitung bekannt ist. wird angewendet, um die Corticalis-Schraube (z.B. mit einem Kerndurchmesser von 3 mm) aus kohlenstoffaserverstärktem PAEK (Poly-Aryl-Ether-Ketone) herzustellen. Eine spezielle Variante sieht vor, kohlenstoffaserverstärktes PEEK (Poly-Ether-Ether-Ketone) einzusetzen. Die Faserorientierungsverteilung und die mechanischen Eigenschaften der Schraube werden charakterisiert und zu den Prozeßparametern des Herstellverfahrens in bezug gebracht.

Die Bruchlast der im Fließpreßverfahren hergestellten Schrauben liegt im Bereich zwischen 3000 und 4000 N, das maximale Torsionsmoment zwischen 1 und 1,5 Nm, wobei der maximale Verdrehwinkel nach ISO-Norm 6475 bis zu 370° beträgt. Die Schrauben besitzen einen vom Kopf zur Spitze hin abnehmenden E-Modul und sind als homoelastisch zum Knochen zu bezeichnen.

Die Natur wendet in ihren Strukturen sehr häufig das Prinzip der Faserverstärkung an. Es ist deshalb aus Gründen der Strukturkompatibilität vorteilhaft, medizinische Implantate ebenfalls als Faserverbundteile zu gestalten. Insbesondere im Bereich der Osteosynthese-Technik sind Entwicklungen erforderlich, um konventionelle Stahl-Osteosyntheseplatten durch weniger rigide Implantate aus Faserverbundwerkstoffen zu ersetzen. Gerade im Zusammenhang mit Osteosyntheseplatten wirkt sich die erfindungsgemäße Ausbildung vorteilhaft aus. Ein solches Osteosynthese-System hat gegenüber einem konventionellen Stahl-Implantat zahlreiche Vorteile. Zum einen ist die Homoelastizität zum Knochen gegeben und deshalb eine angepaßte Lasteinleitung in den Knochen möglich, zum andern ist die Röntgentransparenz und Kernspintomographie möglich. Weiters ergibt sich durch die erfindungsgemäßen Maßnahmen eine kostengünstige Fertigung in einem Warmumformverfahren. Was zusätzlich zählt, ist die Tatsache, daß solcherart ausgebildete Bauteile unproblematisch sind bei Nickel-Allergien.

Bei den Forschungsarbeiten auf diesem Gebiet wurde festgestellt, daß erst durch den Einsatz von Knochenschrauben aus kohlenstoffaserverstärkten Thermoplasten und in diesem Zusammenhang durch die erfindungsgemäßen Hersteilverfahren eine optimale Variante geschaffen werden konnte. Basierend auf dem dabei entwickelten Fließpreßverfahren wurden Knochenschrauben aus kohlenstoffaserverstärktem PEAK hergestellt und charakterisiert.

Beim Fließpressen von Metallteilen wird das Werkstück in der Regel bei Raumtemperatur mittels eines Stempels in eine Form gepreßt. Es gehört damit zu den sogenannten Durchdrückverfahren nach DIN 8583. Für die Verarbeitung der faserverstärkten Thermoplaste wurde das Verfahren dahingehend verändert, daß der Rohlingkörper nicht bei Raumtemperatur, sondern oberhalb der Schmelztemperatur des Matrix-Werkstoffes umgeformt wird.

Als Rohling für die Schraubenherstellung dienen kohlenstoffasen/erstärkte PAEK-Rundstäbe 7 (Fig. 1), M, welche ein Faservolumengehalt von mehr als 50%, vorteilhaft 60% haben, wobei bezüglich der Faserorientierung zwei unterschiedliche Rohling-Typen verwendet wurden, und zwar einerseits Rohlinge mit einer rein achsparallelen Faserorientierung und andererseits Rohlinge mit einer zwischen 0 und ± 90° liegenden Faserorientierung.

Ein Rohlingkörper wird im beheizten Fließpreßwerkzeug 8 (Erwärmungsstufe) auf die Umformtemperatur (z.B. 350 - 450°C) erwärmt, wobei die Erwärmung auch in aufeinander folgenden Erwärmungsstufen 9 und 10 (Fig. 4) erfolgen kann. Der Rohling 7 wird also in die erste Erwärmungsstufe 9 eingebracht, dort entsprechend vorgewärmt, in der Stufe 10 weiter erwärmt und dann im Bereich der Stufe 11 in der Negativform umgeformt. Mittels des Stempels 1-2 wird der Rohling 7 in die Negativform (Formmulde) 13 eingepreßt und erhält dort die endgültige Form. Die Preßgeschwindigkeit kann dabei im Bereich zwischen 2 und 80 mm/s liegen. Der Preßdruck wurde bei verschiedenen Versuchen mit 120 MPa bemessen. Während einer darauffolgenden Nachdruckphase (Preßdruck ist annähernd 90 MPa) wird das Werkzeug mit Druckluft unter die Glasübergangstemperatur von PAEK (143°C) gekühlt. Nach dem Öffnen des Fließpreßwerkzeuges kann die fertige Corticalis-Schraube entnommen werden.

Bei einer nachfolgenden Analyse einer so hergestellten Schraube hat sich gezeigt, daß jeweils optimale Werte erzielt werden können. Dies ergibt sich aus dem hohen Faseranteil, dem Einsatz von Endlosfasern und dem ganz speziellen Warmumformverfahren zur Herstellung der Schraube. Wie aus Fig. 2 ersichtlich ist, richten sich die Fasern im Bereich des Kopfes 2 der Schraube 1 überwiegend in Richtung der Schraubenachse aus. Im Bereich der Schraubenspitze folgen die Fasern im Randbereich der Schraubenkontur (also dem Gewindeverlauf), während in der Kernzone eine zufällig verteilte Faserorientierung vorherrscht.

Bezüglich der mechanischen Eigenschaften ist festzuhalten, daß der Mittelwert der Zugfestigkeit der Corticalis-Schrauben etwa 460 N/mm2 beträgt. Die höchsten Zugfestigkeiten wurden mit Schrauben erzielt, die bei hohen Umformgeschwindigkeiten (ungefähr 80 mm/s) und hohen Rohlingtemperaturen (ca. 400°C) hergestellt wurden. Die Torsionsfestigkeit von Schrauben, die aus Rohlingen mit achsparalleler Faserorientierung hergestellt wurden, ist im Schnitt 18% höher als bei Schrauben aus 0°-/± 45°-faserorientierten Rohlingen. Die Maximalwerte wurden bei Schrauben gemessen, die bei vergleichsweise tiefen Temperaturen (380°C) und tiefen Umformgeschwindigkeiten (2 mm/s) hergestellt wurden. Der Elastizitätsmodul in Schraubenlängsrichtung ist nicht konstant, sondern nimmt zur Spitze hin stark ab. Die E-Moduli variieren zwischen 5 und 23 GPa, wobei Schrauben, die aus Rohlingen mit einer 0°-Faserorientierung hergestellt wurden, tendenziell steifer sind. Dies ist auch eindeutig dem schematischen Diagramm nach Fig. 3 zu entnehmen. Die von der Diagrammlinie dargestellte Steifigkeit nimmt in Richtung des Schraubenkopfes zu, wobei gerade in einem bestimmten Bereich auf die Länge des mit dem Gewinde versehenen Schaftes 5 gesehen ein Knick in dieser Linie gegeben ist. Gerade in diesem Bereich, wie auch der Fig. 2 entnommen werden kann, endet die im Kernbereich vorgesehene achsparallele Faserorientierung.

Am Beispiel einer Corticalis-Schraube ist aufgezeigt worden, daß durch Fließpressen von langfaserverstärkten Thermoplasten in einem Warmumformverfahren auch Bauteile mit komplexen Geometrien hergestellt werden können. Die Faserorientierungsverteilung als die bestimmende Größe für die mechanischen Eigenschaften läßt sich durch geeignete Wahl der Faserorientierung im Rohling in gewissen Grenzen steuern. Die übrigen untersuchten Prozeßparameter (Umformgeschwindigkeit und Umformtemperatur) haben einen geringeren Einfluß auf das Fließpreß-Resultat.

Die Zugfestigkeit von fließgepreßten PAEK-Faserverstärkten Schrauben liegt im Schnitt etwa 30% unter derjenigen vergleichbarer Stahlschrauben. Eine durchschnittliche Bruchkraft von 3200 N ist für Osteosynthese-Anwendungen ausreichend, da eine entsprechende Schraube schon bei einer Zugkraft von 800 - 1300 N aus dem Knochen herausgezogen wird.

Die ISO-Norm 6475 verlangt für Stahlschrauben mit vergleichbaren Dimensionen ein minimales Bruchdrehmoment von 4,4 Nm und einen Torsionswinkel von mindestens 180°. Solche Vorgaben können mit Schrauben aus faserverstärkten Thermoplasten nicht erfüllt werden (maximal 1,3 Nm). Versuche haben allerdings gezeigt, daß ein Überdrehen und damit eine Zerstörung der Schraube beim Eindrehen in den Knochen ausgeschlossen ist, da das Gewinde im Knochen schon bei einem Drehmoment von ca. 0,8 Nm zerstört wurde. Der langsame Abfall der Restfestigkeit nach dem Primärversagen würde auch noch nach einem Bruch ein Ausdrehen der beschädigten Schraube aus dem Knochen erlauben.

Mit einem Elastizitätsmodul zwischen 5 und 23 GPa ist die fließgepreßte Corticalis-Schraube in ihrem elastischen Verhalten dem Knochen ähnlich. Die Steifigkeit in Längsrichtung nimmt zur Spitze hin deutlich ab (abfallender Steifigkeitsgradient). Im eingeschraubten Zustand liegt damit der steife Teil der Schraube (Kopfbereich) cortikalisnah und somit an der steifsten Stelle des behandelten Knochens Mit einer solchen Steifigkeitsverteilung kann eine weitgehend an die Knochenstruktur angepaßte Krafteinleitung erreicht werden.

Mit den hier beschriebenen Verfahren nach der Erfindung ist erstmals die Möglichkeit geschaffen worden, Bauteile aus faserverstärkten Thermoplasten, welche z.B. eine besondere Ausbildung eines Gewindes, eines Kopfes, einer Form usw. haben, in einem Warmumformverfahren herzustellen und über die Materialeigenschaften, insbesondere die exakte Ausrichtung von Fasem, eine zum Anwendungsbereich kompatible Konstruktion zu erreichen.

In der vorstehenden Beschreibung wurde von einem Fließpreßverfahren ausgegangen, welches praktisch nur in einer Richtung wirksam ist. Der Rohling wird dabei auf eine entsprechende Temperatur (teigartiger bzw. honigartig fließender Zustand) gebracht und dann in die Negativform 13 eingepreßt. Es ist auch möglich, gerade bei der Herstellung von streifen-, schienen- oder plattenartigen Teilen, aber auch bei schraubenartigen oder sonstigen Verbindungselementen und auch bei besonderen Formen von Teilen oder bei bestimmter Ausgestaltung von Bolzen usw., ein Gegentaktfließpreßverfahren anzuwenden. Es kann dann unter Umständen durch mehrfaches Hin- und Herpressen, also durch ein- oder mehrfache Bewegungsumkehr der Preßrichtung, eine gewünschte Faserorientierung und Faserverteilung erzielt werden. Weitere Details dazu werden noch anhand der Fig. 6 und 7 näher erläutert. Das Gegentaktfließpreßverfahren kann gerade dann von besonderer Bedeutung sein, wenn im entsprechenden Teil beispielsweise Sacklöcher, Durchgangsöffnungen, Einbuchtungen oder besondere Formgebungen vorgesehen sind. Dann kann der spezielle Verlauf der Fasern beeinflußt und somit der herzustellende Bauteil gerade in jenem Bereich besonders verstärkt ausgeführt werden, in welchem die besondere Verstärkung eben notwendig ist.

Als Beschichtung bei der Anwendung der erfindungsgemäßen Verfahren wird der Einsatz von Kohlenstoff oder Graphit vorgesehen. Diese Beschichtungen oder Trennmittel werden bisher praktisch nur für den Metallbereich und nicht für Kunststoffe benutzt. Hier ergeben sich zusätzliche Voneile. da Graphit im Gegensatz zu den üblichen Trennmitteln für Kunststoffe biokompatibel ist.

In Fig. 2 ist eine in axialer Ausrichtung gesehen nur kurze Öffnung für einen Angriff 3 vorgesehen. Es ist auch möglich, hier ein entsprechend tieferes Sackloch oder aber auch eine axial durchgehende Öffnung vorzusehen, um ein entsprechendes Drehwerkzeug einzusetzen. Dadurch könnte zusätzlich zu den bereits vorhandenen Werten bei der Torsionsfestigkeit ein höheres Eindrehmoment überwunden werden, da ein entsprechendes Werkzeug in entsprechend lange Einsteckkanäle eingesetzt werden kann. Da die Herstellung einer solchen Schraube im erfindungsgemäßen Fließpreßverfahren erfolgt, ist diese zusätzliche Formgebung problemlos möglich

Gerade bei Schienen oder Platten können ebenfalls Durchgangsöffnungen, Einbuchtungen, Sacklöcher usw. vorgesehen werden, welche dann speziell von den Fasern umgeben sind.

Die Faserorientierung in der Schraube 1 nach Fig. 2 oder in einem entsprechend anderen Bauteil für einen anderen Einsatzbereich ist grundsätzlich differenziert zu betrachten. Gerade durch die Maßnahmen der erfindungsgemäßen Verfahren ist es möglich, für jeden speziellen Einsatzzweck eine optimale Faserorientierung am fertiggestellten Bauteil zu ermöglichen. Besonders bei einem hohen Faseranteil von mehr als 50 Vol-% und beim Einsatz von Endlosfasern ergeben sich in vielen Bereichen der Technik. insbesondere im Bereich von Verbindungselementen und im Bereich der Medizintechnik besonders wirkungsvolle Varianten.

In Fig.6 ist in schematischer Darstellung ein Gegentaktfließpreßverfahren gezeigt, wobei die aufeinanderfolgenden Verfahrensschritte I bis IV ersichtlich sind. Beim Schritt I wird der Rohling 7 in eine Erwärmungsstufe (Abschnitt 9,10) eingesetzt und dort auf die Umformungstemperatur aufgeheizt. Beim Schritt II wird der Rohling in Pfeilrichtung 16 in die Negativform 13 hineingepreßt. Beim Schritt III wird der bereits einmal umgeformte Rohling 7 wieder in entgegengesetzter Richtung (Pfeilrichtung 17) zurückgepreßt. Beim Schritt IV wird dann der zwei- oder mehrfach umgeformte Rohling zu dem fertigen Bauteil endverdichtet, abgekühlt und dann entformt.

Mittels in die Negativform 13 eingesetzter oder diese durchdringender Bolzen 15 können Bauteile mit Durchgangsöffnungen 14 hergestellt werden, wobei gerade durch das Gegentaktfließpreßverfahren der Rohling mehrfach an diesen Bolzen 15 vorbeigedrückt wird. Es ergibt sich hier also ein ganz spezieller Verlauf der Fasern 6, wie dies auch aus Fig. 7 zu ersehen ist. Die gleiche oder ähnliche Wirkung ergibt sich auch, wenn an den Längs- und/oder Seitenbegrenzungen des als Montageteil 18 ausgebildeten Bauteiles vorstehende Abschnitte vorhanden wären. In den üblicherweise geschwächten Zonen A ergibt sich so eine dichtere Anordnung der Fasern 6, so daß in diesen Zonen die gleiche Festigkeit oder Steifigkeit wie in den anderen Bereichen B eines solchen Bauteiles ergibt.

Eine solche Ausführung eines Bauteiles eignet sich in hervorragender Weise für Osteosyntheseplatten, welche dann z.B. im Zusammenwirken mit einer nach den erfindungsgemäßen Verfahren hergestellten Schraube eingesetzt werden kann. Die gleichen Vorteile der Biokompabilität treffen dann natürlich auch auf diese Platten zu, wobei außerdem die Festigkeit und Steifigkeit in keiner Weise gegenüber den bisher hauptsächlich eingesetzten Platten aus rostfreiem Stahl zurücksteht.

Beim Gegentaktfließpressen sind verschiedene zusätzliche Parameter möglich, durch welche die Vorherbestimmbarkeit des Faserverlaufes und somit der Anpassung der Festigkeit und Steifigkeit auf die Gestalt des Bauteiles noch weiter verbessert werden kann. So können die Anzahl der Takte und Gegentakte, die Taktlänge, die Taktgeschwindigkeit, der Druck und der Gegendruck eingestellt werden. Die Schritte II und III können nach Belieben wiederholt werden, wobei bei jedem Takt und Gegentakt die Taktweglänge neu gewählt werden kann. Die Zentrierung des Bauteiles beim Schritt IV muß nicht unbedingt erfolgen. Alle Parameter konnen in den Schritten II bis IV beliebig variiert werden.

Die Endlosfasern werden bei einem solchen Verfahren nicht übermäßig beansprucht, so daß sie nicht mehrfach brechen. Der Übergang von Stellen mit stark gerichteten Fasem und Stellen mit homogener Faserverteilung ist kontinuierlich. Das Verfahren erlaubt, im Gegensatz zu einer bekannten Laminiertechnik auch nicht-blechförmige Bauteile herzustellen. Es werden Geometrien ermöglicht, welche sonst nur im Spritzgußverfahren vorkommen. Dabei werden durch die Erfindung sogar wesentlich höhere Festigkeiten erreicht. Es ist damit auch möglich geworden, Bauteile mit Löchern, Hinterschneidungen usw. herzustellen. Es ist möglich, die Faserorientierung so zu optimieren, daß die Kapazität derselben, z.B. in Bezug auf die mechanischen Eigenschaften, voll ausgenutzt werden können. Das Verfahren erlaubt eine Composite-Verarbeitung, die der Enlosfaserverstärkung gerecht wird. In einem Bauteil kommen Stellen mit isotropen und anisotropen Eigenschaften nebeneinander vor, ohne daß eine Grenzfläche vorhanden ist. Da Grenzflächen und Nahtstellen auch Schwachstellen sind, wird durch die Erfindung u.a. auch die Anfälligkeit des Bauteiles auf Ermüdung verringert.

Bei dem hier beschriebenen Gegentaktfließpreßverfahren sind noch weitere Varianten denkbar. So könnte ein Taktvorgang nicht nur in einer Richtung ausgeführt werden, sondern auch unter Verwendung von zwei oder drei Hauptachsen. Weiteres wäre es denkbar, die in Fig. 6 gezeigten Stifte erst nach der Homogenisierung des Rohlings, also nach einem oder mehreren Schritten II oder III einzuschieben. Auch denkbär wäre ein bereits homogenisierter Rohling, der also schon in einer Vorstation ein oder mehrfach umgeformt worden ist.

Es ist auch möglich, Rohlinge einzusetzen, welche aus in deren Längsrichtung verlaufenden Schichten unterschiedlicher Faserorientierung bestehen. Es wäre auch denkbar, einen Rohling (auch unter Fertigung von vorerst Stangenmaterial beliebigen Querschnittes) aus mehr als einem Polymerverbund einzusetzen. In einem solchen Falle könnte der Rohling aus mehreren Schichten mit unterschiedlichem Matrixwerkstoff und unterschiedlicher Anordnung und/oder unterschiedlichem Vol%-Anteil und/oder unterschiedlichem Fasermaterial und/oder unterschiedlicher Länge der Fasern bestehen. Wenn Endtosfasern eingesetzt werden, dann weisen diese in der Regel eine Länge auf, welche wenigstens der Länge des Rohlings 7 entspricht, wie er in Anpassung an den fertigen Bauteil vom Stangenmaterial abgetrennt wird.

## Patentansprüche

1. Verfahren zur Herstellung von Bauteilen aus faserverstärkten Thermoplasten, wobei vorerst ein aus Kurz-. Lang- und/oder Endlosfaser (6) und einem Thermoplast gebildeter Rohling (7) vorgefertigt und dieser Rohling (7) in einem Warmumformverfahren unter Druck in einer Negativform in die endgültige Gestalt des Bauteiles gebracht wird, dadurch gekennzeichnet, daß der Rohling (7) zunächst in einer Erwärmungsstufe auf Umformtemperatur erwärmt und dann durch Fließpressen in die Negativform (13) eingepreßt wird.

2. Verfahren nach Anspruch 1, wobei der Faseranteil aus mehr als 50 Vol- % und zumindest überwiegend aus Endlosfasern besfeht und die Bauteile auf Zug, Biegung und/oder Torsion beansprucht werden.

3. Verfahren nach Anspruch 1 oder 2. dadurch gekennzeichnet, daß der Rohling (7) als Stangenmaterial vorgefertigt und vor dem Warmumformverfahren in für den endgültigen Bauteil erforderliche Längen zugeschnitten wird.

4. Verfahren nach Anspruch 1 oder 2, dadurch gekennzeichnet, daß Endlosfasem (6) in einer Länge eingesetzt werden, welche wenigstens der Länge des Rohlings für den endgültigen Bauteil entspricht.

5. Verfahren nach einem der Ansprüche 1 bis 4, dadurch gekennzeichnet, daß ein Rohling (7) aus in dessen Längsrichtung verlaufenden Schichten unterschiedlicher Faserorientierung umgeformt wird.

6. Verfahren nach einem der Ansprüche 1 bis 4, dadurch gekennzeichnet, daß ein Rohling (7) aus mehr als einem Polymerverbund, z.B. mit mehreren Schichten mit unterschiedlichem Matrixwerkstoff und unterschiedlicher Anordnung und/oder unterschiedlichem Vol%-Antei und/oder unterschiedlichem Fasermaterial und/oder unterschiedlicher Länge der Fasern, umgeformt wird.

7. Verfahren nach einem der Ansprüche 1 bis 6, dadurch gekennzeichnet, daß der Rohling (7) durch ein Gegentaktfließpreßverfahren in den endgültigen Bauteil umgeformt wird,

8. Verfahren nach einem der Ansprüche 1 bis 7, dadurch gekennzeichnet, daß Rohling (7) in der Erwärmungsstufe auf eine Umformtemperatur von 350 - 450°C erwärmt und dann in die Negativform (13) eingepreßt wird, wobei während einer Nachdruckphase eine Abkühlung unter die Glasübergangstemperatur des Thermoplasts von z.B. 143°C erfolgt.

9. Verfahren nach einem der vorhergehenden Ansprüche, dadurch gekennzeichnet, daß beim Warmumformverfahren als Trennmittel Kohlenstoff oder Graphit eingesetzt wird.

10. Verfahren nach einem der vorhergehenden Ansprüche, dadurch gekennzeichnet, daß ein Rohling (7) aus mit Kohlenstoffasern (6) verstärktem PAEK (Poly-Aryl-Ether-Ketone) verarbeitet wird.

11. Verfahren nach einem der Ansprüche 1 bis 10, dadurch gekennzeichnet, daß bei zumindest einem Anteil an Endlosfasern (6) diese im Rohling (7) achsparallel verlaufen.

12. Verfahren nach einem der Ansprüche 1 bis 11, dadurch gekennzeichnet, daß bei zumindest einem Anteil an Fasern (6) diese im Rohling (7) eine Ausrichtung von 0 bis zu 90° aufweisen.

13. Verfahren nach einem der Ansprüche 1 bis 12, dadurch gekennzeichnet, daß die Fasern (6) eine Länge von mehr als 3 mm aufweisen.

14. Verfahren nach einem der Ansprüche 1 bis 13, dadurch gekennzeichnet, daß die Fasern beim Fließpressen oberflächendeckend vom Matrixmaterial umschlossen werden.

15. Verfahren nach einem der Ansprüche 1 bis 14, dadurch gekennzeichnet, daß die Preßtemperatur und die Preßgeschwindigkeit als Variable zur Veränderung der Lage und Ausrichtung der Fasern im fertigen Bauteil eingestellt werden.

16. Verfahren nach einem der Ansprüche 1 bis 15, dadurch gekennzeichnet, daß die Bauteile bei der Warmumformung eine zusätzliche Oberflächenversiegelung erhalten.

17. Bauteil aus faserverstärkten Thermoplasten, hergestellt nach einem Verfahren gemäß wenigstens einem der Ansprüche 1 bis 16, gekennzeichnet durch Bereiche mit durch einen vorherbestimmten Verlauf der Fasern lokal vorherbestimmten Steifigkeiten und Festigkeiten, wobei der Bauteil als Verbindungselement mit einem Angriffsende für ein Werkzeug und einem Gewindeschaft (5) ausgeführt ist und wobei die Steifigkeit des Verbindungselementes durch unterschiedliche Faserorientierung vom Angriffsende zum freien Ende hin variiert.

18. Bauteil nach Anspruch 17, dadurch gekennzeichnet, daß die Fasern (6) vom Angriffsende her bis über die unmittelbar daran anschließenden Gewindegänge (4) zumindest annähernd parallel zur Mittelachse des Bauteiles verlaufen, wogegen die Fasern (6) im restlichen Gewindeabschnitt oberflächennah der Gewindekontur in Achsrichtung des Bauteiles folgen, im Kembereich dieses Abschnittes jedoch eine zum freien Ende hin zunehmend zufällig verteilte Faserorientierung vorgesehen ist.

19. Bauteil nach den Ansprüchen 17 und 18, dadurch gekennzeichnet, daß die Steifigkeit des Bauteiles durch unterschiedliche Faserorientierung vom Angriffsende her gesehen zum freien Ende hin stufenförmig oder kontinuierlich abnimmt.

20. Bauteil nach einem der Ansprüche 17 bis 19, dadurch gekennzeichnet, daß im Bauteil wenigster ein Sackloch oder eine Durchgangsöffnung, z.B. zum Einsatz eines Drehwerkzeuges oder zum Durchführen von Befestigungsmitteln, vorgesehen ist.

21. Bauteil nach Anspruch 20, dadurch gekennzeichnet, daß das Sackloch oder die Durchgangsöffnung bei der Herstellung des Bauteiles eingeformt ist.

22. Bauteil nach einem der Ansprüche 17 bis 21, dadurch gekennzeichnet, daß der Bauteil als für den medizinischen Einsatz strukturkompatible Corticalis- oder Spongiosa-Schraube ausgebildet ist.

23. Bauteil nach Anspruch 17, dadurch gekennzeichnet, daß dieser als streifen- oder plattenförmiger Montageteil (18) mit einer oder mehreren Durchgangsöffnungen (14) und/oder über die Längs- und/oder Seitenbegrenzungen vorstehenden Abschnitten ausgebildet ist, wobei die Steifigkeit und Festigkeit über dessen ganze Länge und/oder Breite und/oder Durchmesser vorherbestimmt ist.

24. Bauteil nach den Ansprüchen 17 oder 23, dadurch gekennzeichet, daß der Bauteil ausgebildet, als Montageteil (18), durch dichtere Anordnung von Fasern (6) im Bereich von Durchgangsöffnungen (14) und/oder vorstehenden Abschnitten in diesen üblicherweise geschwächten Zonen die gleiche Festigkeit und Steifigkeit aufweist wie in anderen Bereichen des Bauteiles.

25. Bauteil nach den Ansprüchen 17, 23 oder 24, dadurch gekennzeichnet, daß der Bauteil als Osteosyntheseplatte, z.B. für den Einsatz mit einer Corticalis- oder Spongiosa-Schraube, ausgebildet ist.

## Claims

1. A process for manufacturing components made of fiber reinforced thermoplastic materials, wherein initially a blank (7) formed of short, long and/or continuous fibers (6) and a thermoplastic material is prefabricated and said blank (7) is formed into the final shape of the component in a thermoforming process under pressure in a female mold, characterized by the step of initially heating said blank (7) to forming temperature in a heating stage and then pressing it into the female mold (13) by a press flow molding operation.

2. The process according to claim 1, wherein the fiber content exceeds 50% by volume and contains at least predominantly continuous fibers, and wherein the components are subjected to tension, bending and/or torsion loads.

3. The process according to claim 1 or 2, characterized by the step of prefabricating the blank (7) as a rod material and cutting it to the length necessary for the final component prior to the thermoforming process.

4. The process according to claim 1 or 2, characterized by using continuous fibers (6) of a length corresponding at least to the length of the blank for the final component.

5. The process according to any one of the claims 1 to 4, characterized by the step of deforming a blank (7) from longitudinally extending layers of differing fiber orientation.

6. The process according to any one of the claims 1 to 4, characterized by the step of deforming a blank (7) from more than one polymeric composite comprising, for example, several layers of different matrix material and different arrangement and/or different percentage by volume and/or different fiber material and/or different fiber length.

7. The process according to any one of the claims 1 to 6, characterized by the step of deforming the blank (7) into the final component using a push-pull press flow molding technique.

8. The process according to any one of the claims 1 to 7, characterized by the step of heating the blank (7) to a deformation temperature of between 350 and 450°C in the heating stage and then pressing it into the female mold (13), a dwell pressure stage effecting cooling to a temperature below the glass transition temperature of the thermoplastic material of, for example, 143°C.

9. The process according to any one of the preceding claims, characterized by using carbon or graphite as release agent in the thermoforming process.

10. The process according to any one of the preceding claims, characterized by processing a blank (7) from carbon fiber (6) reinforced PEK (polyether ketone).

11. The process according to any one of the claims 1 to 10, characterized in that in at least one fraction of the continuous fibers (6) said fibers extend parallel to the axis in the blank (7).

12. The process according to any one of the claims 1 to 11, characterized in that in at least one fraction of the fibers (6) said fibers have an orientation of between 0 and 90° in the blank (7).

13. The process according to any one of the claims 1 to 12, characterized in that the fibers (6) have a length of more than 3 mm.

14. The process according to any one of the claims 1 to 13, characterized in that during press flow molding the matrix material encloses the fibers, covering the complete surface.

15. The process according to any one of the claims 1 to 14, characterized in that the press temperature and the press rate are adjusted as a variable to the variation of the position and orientation of the fibers in the finished component.

16. The process according to any one of the claims 1 to 15, characterized by providing the components with an additional surface seal during the thermoforming process.

17. A component of fiber reinforced thermoplastic materials, manufactured according to a method as claimed in at least one of the claims 1 to 16, characterized by zones of stiffness and strength values predetermined locally by a predetermined fiber orientation, wherein said component is constructed as a fastening element having an end for engagement with a tool and a threaded shank (5), and wherein the stiffness of said fastening element varies on account of differing fiber orientation from the tool engaging end to the free end.

18. The component according to claim 17, characterized in that the fibers (6) extend at least approximately parallel to the component's center line from the tool engaging end up to and beyond the immediately adjoining threads (4), whilst the fibers (6) in the remaining threaded section, extending close to the surface, follow the thread contour in the component's axial direction, while, however, in the core zone of said section a fiber orientation is provided which is increasingly randomly distributed towards the free end.

19. The component according to claims 17 and 18, characterized in that, on account of differing fiber orientation, the stiffness of said component decreases in steps or continuously as seen looking from the tool engaging end towards the free end.

20. The component according to any one of the claims 17 to 19, characterized in that said component includes at least one blind-end hole or one through hole, for example, for use of a rotary tool or for passing fastening elements therethrough.

21. The component according to claim 20, characterized in that said blind-end hole or said through hole is integrally formed during the manufacture of said component.

22. The component according to any one of the claims 17 to 21, characterized in that said component is constructed as a corticalis or spongiosa screw structure-compatible for medical applications.

23. The component according to claim 17, characterized in that it is constructed as a strip- or plate-shaped assembly part (18) having one or several through holes (14) and/or sections projecting over the longitudinal and/or lateral boundaries, with the stiffness and strength being predetermined over its full length and/or width and/or diameter.

24. The component according to claims 17 or 23, characterized in that, owing to a denser arrangement of fibers (6) in the area of through holes (14) and/or projecting sections, the component constructed as assembly part (18) exhibits in these customarily weakened zones the same strength and stiffness as in other zones of the component.

25. The component according to claims 17, 23 or 24, characterized in that the component is constructed as an osteosynthesis plate, for example, for use with a corticalis or spongiosa screw.

## Revendications

1. Procédé de fabrication d'éléments en matière thermoplastique renforcée par des fibres, selon lequel on procède d'abord à la préfabrication d'une ébauche [7] en matière thermoplastique renforcée par des fibres courtes, longues et/ou sans fin [6], puis au moulage de cette ébauche [7] par un procédé de thermoformage sous pression dans une matrice où elle prend la forme définitive de l'élément considéré, ce procédé étant caractérisé par le fait que l'ébauche [7] est portée à la température de formage au cours d'une première phase de réchauffement avant d'être formée par fluage dans la matrice [13].

2. Procédé selon la revendication 1, caractérisé en ce que les fibres sont composées de plus de 50% de fibres sans fin, et les éléments sont soumis à des contraintes de traction, de flexion et/ou de torsion.

3. Procédé suivant l'une des revendications 1 ou 2, caractérisé en ce que l'ébauche [7] préfabriquée se présente sous la forme de barres dont la découpe exécutée avant la phase de thermoformage permet d'obtenir des segments possédant la longueur requise pour réaliser les éléments considérés.

4. Procédé selon l'une des revendications 1 ou 2, caractérisé en ce que les fibres sans fin [6] possèdent une longueur égale ou supérieure à celle de l'ébauche envisagée pour la réalisation des éléments considérés.

5. Procédé suivant l'une quelconque des revendications 1 à 4, caractérisé en ce que le formage par fluage de l'ébauche [7] considérée s'effectue dans le sens de la longueur des couches constitutives de celle-ci, en veillant à ce que l'orientation des fibres diffère d'une couche à l'autre.

6. Procédé selon l'une quelconque des revendications 1 à 4 caractérisé en ce qu'une ébauche [7] ne puisse être le produit d'une seule polymérisation, que cette ébauche doive se composer de plusieurs couches dont la matière première présente un agencement et/ou une composition différente et/ou comporte des fibres de qualité et/ou de longueur différente.

7. Procédé suivant l'une quelconque des revendications 1 à 6 caractérisé en ce que l'ébauche [7] est soumise à un procédé de thermoformage symétrique qui lui fait prendre la forme définitive de l'élément considéré.

8. Procédé suivant l'une quelconque des revendications 1 à 7, caractérisé en ce que, au cours de la phase de réchauffement, l'ébauche [7] est portée à une température comprise entre 350 et 450°C avant d'être formée par fluage dans la matrice [13], puis soumise pendant la phase de maintien en pression à un refroidissement visant à ramener celle-ci à une température inférieure à la température de transition vitreuse de la matière thermoplastique employée, laquelle est de l'ordre de 143°C.

9. Procédé selon l'une quelconque des revendications qui précèdent, caractérisé par l'utilisation de carbone ou de graphite comme agent de séparation lors du procédé de thermoformage.

10. Procédé suivant l'une quelconque des revendications qui précèdent, caractérisé en ce qu'une ébauche [7] est exécutée en PAEC (polyaryléther-cétone) renforcé par fibres de carbone [6].

11. Procédé selon l'une quelconque des revendications 1 à 10, caractérisé en ce qu'une partie au moins de ces fibres sans fin [6] respecte une orientation parallèle à l'axe de l'ébauche [7].

12. Procédé suivant l'une quelconque des revendications 1 à 11 caractérisé, en ce qu'une partie au moins de ces fibres [6] présente une orientation comprise entre 0 et 90° par rapport à l'axe de l'ébauche [7]

13. Procédé selon l'une quelconque des revendications 1 à 12, caractérisé en ce que les fibres [6] possèdent une longueur supérieure à 3 mm.

14. Procédé suivant l'une quelconque des revendications 1 à 13, caractérisé en ce que, lors du formage par fluage, les fibres sont complètement noyées dans la matière première.

15. Procédé selon l'une quelconque des revendications 1 à 14, caractérisé en ce que la température et la vitesse de formage sont considérées comme des variables permettant d'influer sur la position et l'orientation des fibres au sein de l'élément manufacturé.

16. Procédé suivant de l'une quelconque des revendications 1 à 15, caractérisé en ce que, à l'issue de leur thermoformage, les éléments considérés bénéficient d'un scellement de surface complémentaire.

17. Élément en matière thermoplastique renforcée par des fibres, fabriqué selon un procédé découlant au moins de l'une des revendications 1 à 16, caractérisé par la présence de zones dont la contexture des fibres prédétermine la rigidité et la résistance mécanique, et en ce que, d'une part, cet élément pourvu d'un embout d'attaque et d'un pas de vis [5] est utilisé comme pièce de fixation et, d'autre part, la rigidité de cet élément varie de l'embout d'attaque à l'extrémité libre en raison des changements d'orientation des fibres.

18. Élément selon de la revendication 17, caractérisé en ce que les fibres [6] s'étirent presque parallèlement à l'axe de symétrie de l'élément considéré, de l'embout d'attaque aux premières spires du pas de vis [4] tandis que les fibres [6] constitutives du pas de vis, plus proches de la surface des autres spires, ondulent dans l'axe de l'élément considéré et qu'au coeur de cette région de l'élément, l'orientation des fibres devient d'autant plus aléatoire que l'on se rapproche de son extrémité libre.

19. Élément suivant l'une des revendications 17 ou 18, caractérisé en ce que la rigidité de l'élément diminue graduellement ou en continu de l'embout d'attaque à l'extrémité libre sous l'effet des variations d'orientation des fibres constitutives de cet élément.

20. Élément selon l'une quelconque des revendications 17 à 19, caractérisé en ce que cet élément est doté d'un trou borgne ou de passage autorisant le montage d'un outil d'entraînement ou l'exécution d'un moyen de fixation.

21. Élément suivant la revendication 20, caractérisé par la formation du trou borgne ou de passage lors de la fabrication de cet élément.

22. Élément selon l'une quelconque des revendications 17 à 21, caractérisé en ce que cet élément est conçu pour l'emploi médical de vis Corticalis ou Spongiosa compatibles.

23. Élément suivant de la revendication 17, caractérisé par l'utilisation de cet élément comme pièce de montage [18] en forme de plaque ou de patte pourvue d'un ou plusieurs orifices de passage [14] dont la rigidité et la résistance mécanique sont prédéterminées sur toute la longueur, la largeur et/ou le diamètre.

24. Élément selon l'une des revendications 17 ou 23, caractérisé en ce que cet élément utilisé comme pièce de montage [18] présente, grâce à la densité accrue des fibres [6] dans la région des trous de passage [14] et/ou dans les sections qui précèdent, la même rigidité et la même résistance mécanique dans ces zones affaiblies que dans les autres régions de l'élément.

25. Élément suivant l'une quelconque des revendications 17, 23 ou 24, caractérisé par l'utilisation de cet élément comme plaque d'ostéosynthèse posée au moyen de vis Corticalis ou Spongiosa.
